# EUROPEAN PATENT SPECIFICATION

(11) **EP 2 645 101 B1**
(45) Date of publication and mention of the grant of the patent: **08.03.2017**
(21) Application number: 13161539.5
(22) Date of filing: 28.03.2013
(51) Int. Cl.: G01N 33/487

(54) **Test strip reader**
Teststreifenauswertegerät
Lecteur de bande de test

(30) Priority: 30.03.2012 JP 2012078691
(43) Date of publication of application: 02.10.2013
(73) Proprietor: ARKRAY, Inc., Minami-ku Kyoto-shi Kyoto 601-8045 (JP)
(72) Inventor: Fukuda, Kazuo, Kyoto, Kyoto 602-0008, (JP); Nakanishi, Hiroyuki, Kyoto, Kyoto 602-0008, (JP); Hata, Hitoshi, Kyoto, Kyoto 602-0008, (JP)
(74) Representative: MacDougall, Alan John Shaw

(56) References cited:
- EP-A1- 2 107 367
- EP-A1- 2 333 551
- EP-A2- 2 256 495
- WO-A1-2012/035726
- US-A1- 2006 277 048
- US-A1- 2009 282 192

## Description

### BACKGROUND OF THE INVENTION

### 1. Field of the Invention

The present invention relates to a measuring apparatus and a measuring system, and in particular to a measuring apparatus and a measuring system that measure the condition of a user.

### 2. Description of Related Art

In a medical setting in recent years, various items such as blood glucose level, body temperature, pulse rate, and blood pressure have been measured on patients in order to manage their health. The measurement results obtained by such measurement are brought together for each patient and used for a diagnosis by a doctor or the like. Especially, the blood glucose level of a patient needs to be measured several times a day and insulin needs to be administered depending on the measurement results of blood glucose level, and therefore it is important to accurately manage measurement results of blood glucose level for each patient. In view of this, conventionally, a blood glucose measurement management system has been developed that prevents the confusion of blood glucose meters among patients, and accurately manages the measurement results of each patient.

For example, Patent Document 1 discloses a blood glucose measurement management system constituted by a blood glucose level measuring apparatus and a measured data management apparatus that are connected to each other. In the blood glucose measurement management system disclosed in Patent Document 1, the measured data management apparatus reads a serial number stored in the blood glucose level measuring apparatus, and transmits a nickname recorded in association with the read serial number, together with measured data, to the measured data management apparatus. Accordingly, the measured data management apparatus displays the measured data and the nickname on a display unit. As a result, a doctor or the like can easily identify, with the displayed nickname, to whom the measured data belongs.

As described above, use of the blood glucose measurement management system disclosed in Patent Document 1 is considered to prevent confusion in medical settings, since the blood glucose level measuring apparatus is managed with a nickname, instead of a serial name.

### Citation List

### Patent Document

Patent Document 1: WO 2009-081790 A

Further background art is provided in EP 2 333 551 A1, EP 2 107 367 A1, WO 2012/035726 A1 and US 2006/277048.

EP 2 333 551 A1 discloses a blood glucose level measuring apparatus and a measurement data management apparatus which can carry out measurement of the blood glucose level of a plurality of patients and can deal also with an emergency situation. The blood glucose level measuring apparatus includes patient identification means, blood glucose level measuring means, time counting means, display means, control means having a normal measurement mode in which patient identification information and a patient data table are cross-checked with each other and then, if the patient identification information is not found, measurement of the blood glucose level is inhibited and an emergency measurement mode in which carrying out of measurement of the blood glucose level is permitted without acquiring the patient identification information by the identification means.

EP 2 107 367 A1 discloses a code reading device capable of associating various kinds of information with obtained data by connecting the code reading device to a data holding device. A code reading device includes a code reader for reading code information, a memory, and a data processing control portion. The data processing control portion stores code information read by the code reader in a code information storage area of the memory, reads data from a data holding device when the data holding device is connected to the code reading device and stores the data in a data storage area of the memory, and sends to a personal computer the data in the data storage area in a state of being associated with the code information in the code information storage area when the personal computer is connected to the code reading device.

WO 2012/035726 A1 discloses a biological sample data management device, and biological sample measurement devices connected to the biological sample data management device. At least one of the brightness of a display unit and the volume of an operation sound generation unit is set as a first setting value in the biological sample measurement device, this set first setting value is transmitted to the biological sample measurement devices by means of the biological sample data management device, and the brightness of a display unit or the volume of an operation sound generation unit in the biological sample measurement devices is set as a first setting value.

US 2006/277048 is related to a hand-held device for glucose assays with acoustic output to aid visual impaired users.

### SUMMARY OF THE INVENTION

Although a preset nickname is displayed on the display unit of the measured data management apparatus in the above-described blood glucose measurement management system of Patent Document 1, it is impossible to check the nickname set in the blood glucose level measuring apparatus before connecting the blood glucose level measuring apparatus to the measured data management apparatus. Therefore, in order to check whether or not a blood glucose level measuring apparatus is the blood glucose level measuring apparatus that is to be used, it is necessary to connect blood glucose level measuring apparatuses one by one to the measured data management apparatus so as to check their nicknames. As a result, this causes a problem of lowering the working efficiency of a healthcare worker such as a doctor or nurse, especially, in a hospital ward or the like where many blood glucose level measuring apparatuses are used.

In a medical setting, there is also the case where a single measuring apparatus is used for measurement performed on a plurality of patients. In this case, if it were not easy to check whether the measured data is correlated with identification information of a patient, it would take much time for a healthcare worker to check the correspondence. As a result, this also causes a similar problem of lowering working efficiency.

The present invention is a measuring apparatus as defined in Claim 1 of the appended claims. Also provided is a measuring system as defined in Claim 8.

One example of an object of the present invention is to provide a measuring apparatus and a measuring system that solve the above-mentioned problem and enable measured data and identification information that corresponds to the measured data to be easily and reliably correlated with each other.

In order to achieve the above-mentioned object, a measuring apparatus of the present invention is directed to a measuring apparatus for measuring the condition of a measurement subject, including: a measuring unit for obtaining a measurement result; an input unit for receiving input of information and for outputting the received information to a setting unit; recording unit configured to record a plurality of behaviors that can be set in the measuring apparatus, and pieces of measurement subject-specific identification information that are associated with the plurality of behaviors; a setting unit configured to specify a piece of user-specific or medical-facility-specific identification information that is correlated with a measurement result in accordance with external input, and to set the specified piece of identification information and the behavior recorded by the recording unit associated with the specified piece of identification information, as identification information and a behavior that are to be used; and an output unit configured to output the behavior set by the setting unit; wherein the behaviors are (a) signals to allow a user to identify the measuring apparatus from among a plurality of such measuring apparatuses, or (b) signals to allow a user to determine whether or not the measuring apparatus has been set with measurement information of the user.

Since the measuring apparatus of the present invention can output behaviors that can be set, a user of the apparatus, such as a healthcare worker or a patient as a measurement subject, can identify an identification number to be used based on the
output behaviors, and input the identification number into the measuring apparatus. According to the measuring apparatus of the present invention, it is thus possible to easily and reliably correlate measured data with identification information corresponding thereto.

In the measuring apparatus of the present invention, it is preferable that the behaviors be each defined by at least one of sound that is output by the measuring apparatus, screen display that is output by the measuring apparatus, light that is output by the measuring apparatus, and vibration that is output by the measuring apparatus.

Accordingly, setting, for example, different screen display depending on users of the measuring apparatus enables a healthcare worker or a measurement subject such as a patient to easily check the behavior by merely viewing the measuring apparatus. Also, varying the type of light, the type of sound, or the type of vibration that are to be output enables even a measurement subject who has poor eyesight to easily check the behavior. Further, combining two or more of the screen display, the type of light, the type of sound, and the type of vibration that are to be output enables a measurement subject or a healthcare worker to check the behavior not only visually but also auditorily, making more accurate checking possible.

In the measuring apparatus of the present invention, specifically, it is preferable that the setting unit cause the output unit to output the plurality of different behaviors and specify, when any of the behaviors is selected through external input, a piece of identification information that is associated with the selected behavior as the identification information correlated with the measurement result. This case is especially effective if a patient (measurement subject) as the user has poor eyesight and cannot clearly view the identification information displayed on the display screen.

The order of output of the plurality of different behaviors in the setting unit may be determined and set in advance in the setting unit or may be externally added and set in the setting unit.

The measuring apparatus of the present invention preferably has an aspect in which it further includes a communication unit configured to be able to intercommunicate with a communication terminal.

The measuring apparatus having such a configuration can be made easy to use such that the measuring apparatus is activated, for example, by operation of the communication terminal.

Also, the measuring apparatus of the present invention preferably has an aspect in which upon input of a new behavior and identification information that is associated with the new behavior from the communication terminal, the communication unit receives the new behavior and the identification information, and when the communication unit has received the input of the new behavior and the identification information associated with the new behavior, the recording unit records the received new behavior and the identification information associated with the new behavior.

According to the measuring apparatus having such a configuration, the user of the measuring apparatus can add a new behavior that is output from the measuring apparatus or change an existing behavior by operating the communication terminal.

Further, the measuring apparatus of the present invention preferably has an aspect in which the communication unit receives, from the communication terminal, a confirmation instruction for confirmation of the behavior associated with the identification information of the user of the measuring apparatus, and when the communication unit has received the confirmation instruction, the output unit outputs the behavior associated with the identification information of the user of the measuring apparatus.

According to the measuring apparatus having such a configuration, the user can easily confirm the behavior corresponding to his/her own identification information.

Also, the measuring apparatus of the present invention preferably has an aspect in which, when the communication unit has received an identification number specification instruction for instructing the output unit to output the behaviors in a predetermined order so that the setting unit specifies the identification information, the setting unit causes the output unit to output the plurality of different behaviors in the predetermined order.

According to the measuring apparatus having such a configuration, it is possible to automatically and easily confirm the behavior that corresponds to the identification information.

The measuring apparatus of the present invention preferably has an aspect in which it further includes a warning unit configured to output a warning, wherein the communication unit causes the warning unit to output a warning, when having detected change in a communication state with the communication terminal.

Here, the case where the communication unit detects change in a communication state may be the case where communication between the measuring apparatus and the communication terminal becomes increasingly difficult, examples of the case including the case where a signal in the measuring apparatus that has been transmitted from the communication terminal has a radio field intensity that is not more than a threshold, and the case where the signal transmitted from the communication terminal has a radio wave S/N ratio that is not more than a threshold. Note that the measuring apparatus of the present invention may have a configuration such that when the measuring apparatus and the communication terminal are separated from each other by a predetermined distance or more, a warning signal is transmitted to the communication terminal so that a warning sound or the like is output in the communication terminal.

According to the measuring apparatus having such a configuration, a warning is given if the communication terminal and the measuring apparatus are separated from each other and no signal is thus transmitted and received therebetween, making it possible to prevent the measuring apparatus from being misplaced.

In order to achieve the above-mentioned object, a measuring system of the present invention includes a measuring apparatus configured to measure the condition of a measurement subject, and a communication terminal, the measuring apparatus including: a recording unit configured to record a plurality of different behaviors that can be set in the measuring apparatus, and pieces of identification information that are associated with the plurality of behaviors; an output unit configured to output the behaviors; a setting unit configured to specify a piece of identification information that is correlated with a measurement result in accordance with external input, and to set the specified piece of identification information and a behavior associated with the specified piece of identification information, as identification information and a behavior that are to be used; and a communication unit configured to be able to intercommunicate with the communication terminal.

According to the measuring system of the present invention, since the measuring apparatus can output behaviors that can be set, a user of the apparatus, such as a healthcare worker or a patient as a measurement subject, can identify an identification number to be used based on the output behaviors, and input the identification number into the measuring apparatus. According to the measuring system of the present invention, it is thus possible to easily and reliably correlate measured data with identification information corresponding thereto. Also, according to the measuring system of the present invention, the measuring apparatus can be made easy to use such that the measuring apparatus is activated, for example, by operation of the communication terminal.

Further, the measuring system of the present invention preferably has an aspect in which upon input of a new behavior and identification information that is associated with the new behavior from the communication terminal, the communication unit receives the new behavior and the identification information, and when the communication unit has received the input of the new behavior and the identification information associated with the new behavior, the recording unit records the received new behavior and the identification information associated with the new behavior.

According to the measuring system having such a configuration, the user of the measuring apparatus can add a new behavior that is output from the measuring apparatus or change an existing behavior by operating the communication terminal.

Also, the measuring system of the present invention preferably has an aspect in which the communication unit receives, from the communication terminal, a confirmation instruction for confirmation of the behavior associated with the identification information of the user of the measuring apparatus, and when the communication unit has received the confirmation instruction, the output unit outputs the behavior associated with the identification information of the user of the measuring apparatus.

According to the measuring system having such a configuration, the user can easily confirm the behavior corresponding to his/her own identification information.

The measuring system of the present invention preferably has an aspect in which the measuring apparatus further includes a warning unit configured to output a warning, wherein the communication unit causes the warning unit to output a warning, when having detected change in a communication state with the communication terminal.

Here, the case where the communication unit detects change in a communication state may be the case where communication between the measuring apparatus and the communication terminal becomes increasingly difficult, examples of the case including the case where a signal in the measuring apparatus that has been transmitted from the communication terminal has a radio field intensity that is not more than a threshold, and the case where the signal transmitted from the communication terminal has a radio wave S/N ratio that is not more than a threshold. Note that in the measuring system of the present invention, the measuring apparatus may have a configuration such that when the measuring apparatus and the communication terminal are separated from each other by a predetermined distance or more, a warning signal is transmitted to the communication terminal so that a warning sound or the like is output in the communication terminal.

According to the measuring system having such a configuration, a warning is given if the communication terminal and the measuring apparatus are separated from each other and no signal is thus transmitted and received therebetween, making it possible to prevent the measuring apparatus from being misplaced.

As described above, according to the measuring apparatus and the measuring system of the present invention, it is possible to easily and reliably correlate measured data with identification information that corresponds to the measured data.

### BRIEF DESCRIPTION OF THE DRAWINGS

FIG. 1 is a diagram illustrating an external appearance of a measuring apparatus of Embodiment 1 of the present invention.
FIG. 2 is a block diagram illustrating an internal configuration of the measuring apparatus of Embodiment 1 of the present invention.
FIG. 3 is a diagram illustrating a table that is stored in a recording unit of the measuring apparatus of Embodiment 1 of the present invention.
FIG. 4 is a flowchart illustrating a flow of preliminary preparation processing performed in the measuring apparatus of Embodiment 1 of the present invention.
FIG. 5 is a flowchart illustrating a flow of measurement processing performed in the measuring apparatus of Embodiment 1 of the present invention.
FIG. 6 is a diagram illustrating a situation in which a patient as a measurement subject identifies the measuring apparatus to be used for measurement, among a plurality of measuring apparatuses.
FIG. 7 is a diagram illustrating a situation in which when a single measuring apparatus is used for measurement performed on a plurality of measurement subjects, a patient as a measurement subject checks whether or not his/her own measurement information is set in the measuring apparatus.
FIGS. 8A to 8C illustrate situations in which a patient as a measurement subject is performing selection operations based on behaviors from the measuring apparatus, specifically, the figures illustrating states in which different behaviors are output.
FIG. 9 is a block diagram illustrating a configuration of a measuring system including a measuring apparatus of Embodiment 2 of the present invention.
FIG. 10 is a flowchart illustrating a flow of warning processing performed in the measuring apparatus of Embodiment 2 of the present invention.

### DETAILED DESCRIPTION OF THE INVENTION

### Embodiment 1

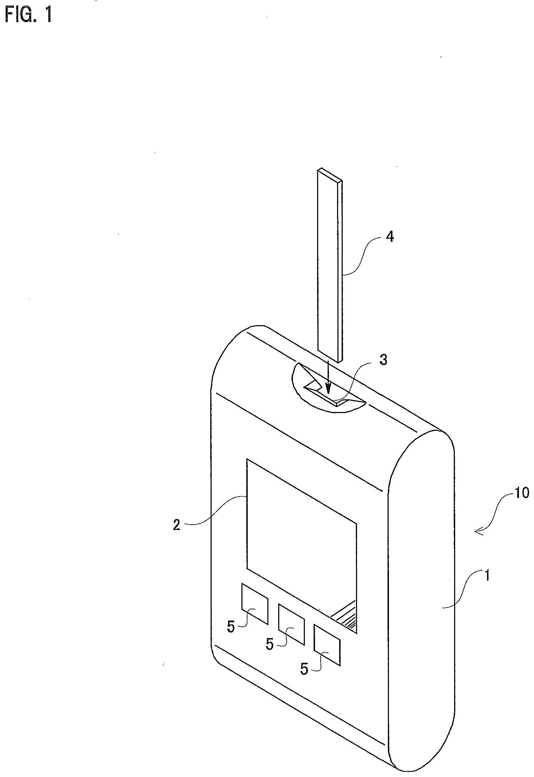

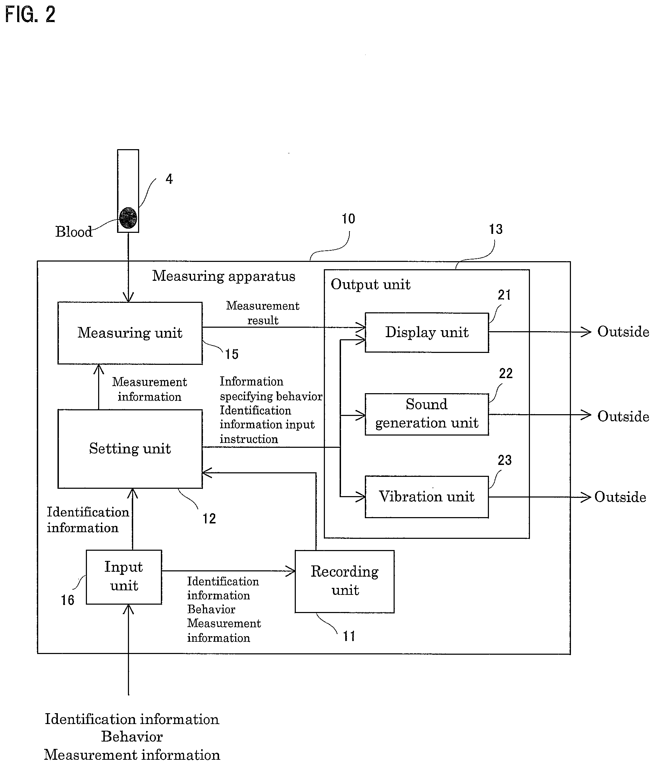

Hereinafter, a measuring apparatus of Embodiment 1 of the present invention will be described with reference to the drawings. FIG. 1 is a diagram illustrating an external appearance of a measuring apparatus 10 of Embodiment 1 of the present invention. FIG. 2 is a block diagram illustrating an internal configuration of the measuring apparatus 10 of Embodiment 1 of the present invention.

### Apparatus Configuration

The overall configuration of the measuring apparatus 10 will first be described, with reference to FIG. 1. As illustrated in FIG. 1, the measuring apparatus 10 of Embodiment 1 of the present invention is a medical device for measuring the condition of a measurement subject, examples of which include a blood glucose meter, a sphygmomanometer, a lactic acid meter, a ketone body measurement device, a thermometer, a urine test paper meter, and a lipid measurement device. In the present Embodiment 1, the measuring apparatus 10 preferably has a size such that it fits in the palm of a hand, thus enabling a patient, a doctor, a nurse, or the like to carry the measuring apparatus 10. Hereinafter, description will be made on the assumption that the measuring apparatus 10 is portable. Specifically, the present Embodiment 1 will describe the case where the measuring apparatus 10 is a portable blood glucose meter for measuring the blood glucose level in the blood of a measurement subject.

As illustrated in FIG. 1, the measuring apparatus 10 includes a display screen 2, a sensor insertion opening 3, and buttons 5 in a main body 1. The display screen 2 is a screen for displaying results of measurement performed by the measuring apparatus 10. A strip-shaped sensor 4 is inserted into the sensor insertion opening 3. An activation switch is provided inside the sensor insertion opening 3, and is turned on upon insertion of the sensor 4 into the sensor insertion opening 3 so as to switch the measuring apparatus 10 to an operating state. Note that the activation switch does not need to be provided at a location inside the sensor insertion opening 3, and may be provided at any desired location in the measuring apparatus 10.

The sensor 4 has a reagent inside and, when blood collected from a measurement subject such as a patient adheres to the sensor 4, the reagent reacts with the blood inside the sensor 4. The measuring apparatus 10, which switches to the operating state upon insertion of the sensor 4 into the sensor insertion opening 3, measures the blood glucose level in the blood that reacted with the reagent within the main body 1 of the measuring apparatus 10. The buttons 5 are used when the measurement subject or a healthcare worker inputs identification information of the measurement subject, date and time information, and the like into the measuring apparatus 10.

Also, the measuring apparatus 10 is configured such that a behavior that corresponds to the measurement subject can be set, and outputs the set behavior according to need. In the present Embodiment 1, the behavior is defined by at least one of sound that is output by the measuring apparatus 10, screen display that is output by the measuring apparatus 10, light that is output by the measuring apparatus 10, and vibration that is output by the measuring apparatus 10.

Specifically, first, examples of the behavior defined by sound include generation of specific sound, change of the sound intensity, change of the tone, change of the sound pattern, and change of the sound length. Examples of the behavior defined by screen display include display of specific characters and number, display of a specific image (such as a picture), change of the color used for display, and change of the brightness of the display screen. Examples of the behavior defined by light include light emission of the buttons 5 and change of the color of the buttons 5. Examples of the behavior defined by vibration include generation of specific vibration, change of the intensity of vibration, change of the vibration pattern, and change of the vibration length. In the present Embodiment 1, the behavior set in the measuring apparatus 10 may be one of the above-mentioned behaviors or a combination of them.

Next, an internal configuration of the measuring apparatus 10 will be described with reference to FIG. 2. As illustrated in FIG. 2, the measuring apparatus 10 includes a recording unit 11, a setting unit 12, and an output unit 13. These components are provided inside the main body 1 illustrated in FIG. 1. Specifically, the recording unit 11 has recorded therein a plurality of behaviors that can be set in the measuring apparatus 10 and pieces of identification information that are associated with the respective behaviors. The output unit 13 outputs the behaviors. The setting unit 12 specifies a piece of identification information that is correlated with a measurement result in accordance with external input, and sets the specified piece of identification information and the behavior associated with the identification information, as identification information and behavior that are to be used.

Since the measuring apparatus 10 having such a configuration can output the behaviors that can be set, a user of the measuring apparatus 10, such as a healthcare worker or a patient as a measurement subject, can identify an identification number to be used based on the output behaviors, and input this identification number into the measuring apparatus. According to the measuring apparatus 10, it is thus possible to easily and reliably correlate measured data and identification information corresponding to the measured data with each other. As a result, this eliminates the need for the healthcare worker to perform a complicated operation as conventionally performed, thus suppressing a reduction in working efficiency

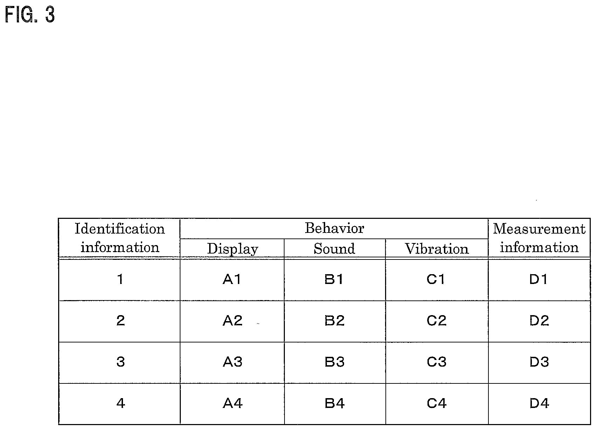

Now, the configuration of the measuring apparatus 10 of Embodiment 1 of the present invention will be described in further detail with reference to FIG. 3, in addition to FIGS. 1 and 2. FIG. 3 is a diagram illustrating a table stored in the recording unit 11 of the measuring apparatus 10 of Embodiment 1 of the present invention.

As illustrated in FIG. 2, the measuring apparatus 10 further includes a measuring unit 15 and an input unit 16, in addition to the recording unit 11, the setting unit 12, and the output unit 13.

The input unit 16, which is connected to the buttons 5, receives input of information through operation of the buttons 5, and outputs the received information to the setting unit 12. In the present Embodiment 1, examples of information whose input is received by the input unit 16 include identification information, behavior, and measurement information. Note that the input of identification information and measurement information is made when, for example, a new measurement subject is registered. The input of behavior is made when, for example, a behavior is associated with identification information of the new measurement subject or when the already registered behavior of the measurement subject is changed.

The identification information is a unique ID (Identifier) that is set for each measurement subject.

Examples of the measurement information include past measurement results that are managed for each measurement subject, and measurement conditions unique to the measurement subject, such as height, weight, and age. In the present Embodiment 1, the measurement information as well as the identification information can be correlated with the measured data.

Also, in the present Embodiment 1, the setting unit 12 can display options for identification information, behavior, and measurement information on the display unit 21 at the time of an input operation. In this case, the user of the measuring apparatus 10, such as a healthcare worker or a measurement subject, does not need to input the actual value into the measuring apparatus 10 via the buttons 5 but only needs to perform selection operations using the buttons 5. In this case, the input unit 16 notifies the setting unit 12 of the fact that a button has been pressed. Upon having been notified, the setting unit 12 determines that information that is, for example, highlighted on the screen has been selected, and obtains the information as input information.

As illustrated in FIG. 3, for example, it is assumed that the measuring apparatus 10 has stored therein in advance a plurality of patterns with respect to aspects of display, types of sound, and types of vibration. In this case, the user such as a healthcare worker or a measurement subject selects, on the screen, a pattern that is a combination of an aspect of display screen (A1 to A4), a type of sound (B1 to B4), and a type of vibration (C1 to C4). The input of the behaviors will end therewith.

The recording unit 11 has stored therein the table illustrated in FIG. 3, and records the input identification information, behavior, and measurement information in association with one another in this table. For example, in the example of FIG. 3, the aspect of display "A1", the type of sound "B1", the type of vibration "C1", and the type of measurement information "D1" are recorded in association with the identification information "1" in the table. Note that although, in the example of FIG. 3, the three items of identification information, behavior, and measurement information are associated with one another, the present Embodiment 1 is not limited to this example, and only the two items of identification information and behavior, for example, may be associated with each other.

In the present Embodiment 1, when the measuring apparatus 10 has switched to the operating state, the setting unit 12 first references the table stored in the recording unit 11, and determines whether or not a plurality of different behaviors and pieces of identification information corresponding to the respective behaviors are recorded in the recording unit 11.

If a plurality of different behaviors and pieces of identification information corresponding to the respective behaviors are recorded in the recording unit 11, the setting unit 12 outputs to the output unit 13 an identification information input instruction for requesting the user of the measuring apparatus 10 to input identification information. Upon having received the identification information input instruction, the output unit 13 can request the user to input the identification information by outputting a behavior such as change in display on the display screen 2, generation of sound, and generation of vibration.

When the user such as a healthcare worker or a measurement subject has input the identification information via the buttons 5 and the input unit 16 in accordance with the identification information input instruction, the setting unit 12 specifies the input identification information and sets it as identification information for use in measurement. Meanwhile, when the user has made selection among the options displayed on the display screen 2 using the buttons 5, the setting unit 12 specifies the selected identification information and sets it as identification information for use in the measurement.

Then, the setting unit 12 references the table in the recording unit 11, and sets the behavior that is associated with the set identification information as behavior to be used, that is, the behavior of the measuring apparatus 10. The setting unit 12 then outputs information that specifies the set behavior to the output unit 13, and then the output unit 13 outputs the set behavior. Meanwhile, if measurement information is associated with the identification information in the table, the setting unit 12 outputs the measurement information associated with the obtained identification information to the measuring unit 15, and sets the output measurement information as a measurement condition.

Note that, if the recording unit 11 has recorded therein a single behavior and a single piece of identification information, the setting unit 12 sets, for example, this single behavior recorded in the recording unit 11 when the measuring apparatus is switched to the operating state (when the measuring apparatus is activated). In this case, the behavior is output from the output unit 13 at the same time when the measuring apparatus switches to the operating state, thus making it possible for the healthcare worker and the measurement subject to easily find out the measuring apparatus to be used.

Upon insertion of the sensor 4 into the sensor insertion opening 3, the measuring unit 15 measures the blood glucose level in the blood that reacted with the reagent. At this time, the measuring unit 15 obtains a measurement result based on the measurement condition set by the setting unit 12 (see FIG. 3), and outputs the obtained measurement result to the output unit 13.

The output unit 13 is provided with a display unit 21, a sound generation unit 22, and a vibration unit 23. Specifically, the display unit 21 is a display panel such as a liquid crystal display panel in the present Embodiment 1, and is capable of displaying the measurement result output by the measuring unit 15 and date and time information on the display screen 2. The sound generation unit 22 is a speaker in the present Embodiment 1, and is capable of outputting sound at the time of, for example, the start and end of measurement. The vibration unit 23 is a vibration unit provided with, for example, a piezoelectric vibration plate, or a vibration motor, and is capable of vibrating the main body 1. The vibration unit 23 can also generate vibration in conjunction with generation of sound made by the sound generation unit 22.

When a predetermined behavior that corresponds to the identification information has been set by the setting unit 12, the output unit 13 performs output according to the set behavior, using at least one of the display unit 21, the sound generation unit 22, and the vibration unit 23. Note that the output unit 13 can also output different behaviors depending on the remaining battery level of the measuring apparatus 10.

### Apparatus Operation

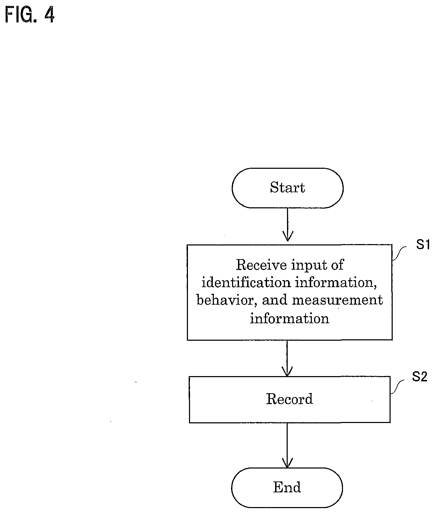

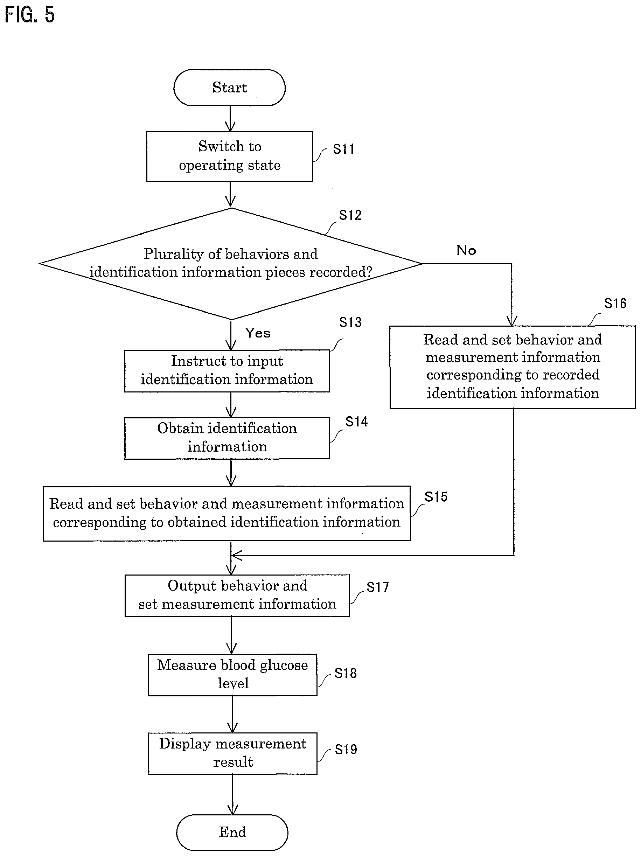

Now, operation of the measuring apparatus 10 in Embodiment 1 of the present invention will be described with reference to FIGS. 4 and 5. FIG. 4 is a flowchart illustrating a flow of preliminary preparation processing performed in the measuring apparatus 10 of Embodiment 1 of the present invention. FIG. 5 is a flowchart illustrating a flow of measurement processing performed in the measuring apparatus 10 of Embodiment 1 of the present invention.

### Apparatus Operation: Preliminary preparation processing

First, the preliminary preparation processing that is performed before measurement will be described with reference to FIG. 4. As illustrated in FIG. 4, when the user such as a healthcare worker or a measurement subject first inputs identification information of the measurement subject, a behavior that is to be set, and measurement information using the buttons 5, the input unit 16 receives the input of these pieces of information (step S1).

Next, the input unit 16 outputs the input identification information and behavior to the recording unit 11, and causes the recording unit 11 to store the identification information and the behavior in association with each other in a table (step S2). Note that the input unit 16 can also output the measurement information to the recording unit 11. In this case, the recording unit 11 also stores the measurement information in association with the identification information and the behavior in the table. In the present embodiment, the input unit 16 may output the measurement information to the setting unit 12, instead of the recording unit 11. In this case, the setting unit 12 outputs the measurement information that corresponds to the input identification information to the measuring unit 15 and sets the output measurement information as a measurement condition.

### Apparatus Operation: Measurement Processing

Next, the measurement processing performed by the measuring apparatus 10 will be described with reference to FIG. 5.

First, when the user inserts the sensor 4 into the sensor insertion opening 3, the activation switch is turned on, and the measuring apparatus 10 is activated. This allows the setting unit 12 to switch the measuring apparatus 10 to the operating state, as illustrated in FIG. 5 (step S11).

Then, the setting unit 12 references the table in the recording unit 11, and determines whether or not a plurality of different behaviors and pieces of identification information corresponding to the respective behaviors are recorded in the recording unit 11 (step S12).

If the result of the determination in step S12 is that a plurality of different behaviors and pieces of identification information corresponding to the respective behaviors are recorded in the recording unit 11 (Yes, in step S12), the setting unit 12 then outputs an identification information input instruction to the output unit 13 (step S13). When step S13 has been executed, in the output unit 13, for example, the display unit 21 displays on the display screen 2 the instruction for prompting the user to input the identification information, the sound generation unit 22 outputs sound, and the vibration unit 23 vibrates the main body 1 of the measuring apparatus 10 in conjunction with the sound.

Next, if the user has input the identification information via the buttons 5 according to the various types of output by the output unit 13, the input unit 16 outputs the input identification information to the setting unit 12, and therefore the setting unit 12 sets the input identification information as identification information to be used (step S14). Also, if the user selects, using a button 5, identification information from the options displayed on the display screen 2, the input unit 16 notifies the setting unit 12 of the fact that the button has been pressed, and therefore the setting unit 12 sets the selected and input identification information as identification information to be used.

Next, the setting unit 12 references the table in the recording unit 11, and reads the behavior that is associated with the identification information set in step S14 and sets the read behavior (step S15). In step S15, the setting unit 12 outputs information that specifies the set behavior to the display unit 21, the sound generation unit 22, and the vibration unit 23 depending on the type of the behavior. Note that if measurement information is associated with the identification information, the setting unit 12 reads this measurement information as well, outputs the read measurement information to the measuring unit 15, and sets the read measurement information as a measurement condition.

When step S15 has been performed, the output unit 13 then outputs the behavior using at least one of the display unit 21, the sound generation unit 22, and the vibration unit 23, depending on the set behavior (step S17).

When step S17 has been performed, the user can then determine whether or not the measuring apparatus 10 is the measuring apparatus 10 that is to be used by determining whether or not the behavior output from the measuring apparatus 10 matches the expected behavior. If the measuring apparatus 10 is the measuring apparatus that is to be used, the user can confirm that they match, using a button 5.

If the user has confirmed, the measuring unit 15 measures the blood glucose level according to the measurement condition set in step S15 (step S18). Thereafter, the measuring unit 15 outputs the measurement result to the display unit 21, and then the display unit 21 displays the measurement result on the display screen 2 (step S19).

On the other hand, if the result of the determination in step S12 is that the recording unit 11 has recorded therein a single behavior and a single piece of identification information (No, in step S12), the setting unit 12 references the table in the recording unit 11 and reads the recorded behavior and measurement information. The setting unit 12 then sets the read behavior (step S16).

If measurement information is associated with the identification information, the setting unit 12 also reads the measurement information, outputs the read measurement information to the measuring unit 15, and sets the output measurement information as a measurement condition. Further in step S16, similarly to step S15, the setting unit 12 outputs information that specifies the set behavior to the display unit 21, the sound generation unit 22, and the vibration unit 23 depending on the type of the behavior.

After the execution of step S16, the above-described steps S17 to S19 are executed, and ultimately the measurement result is displayed on the display screen 2.

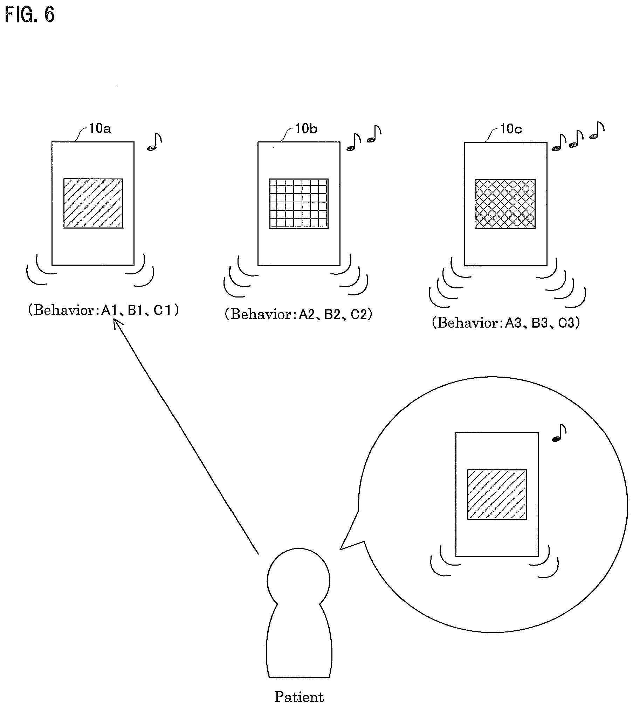

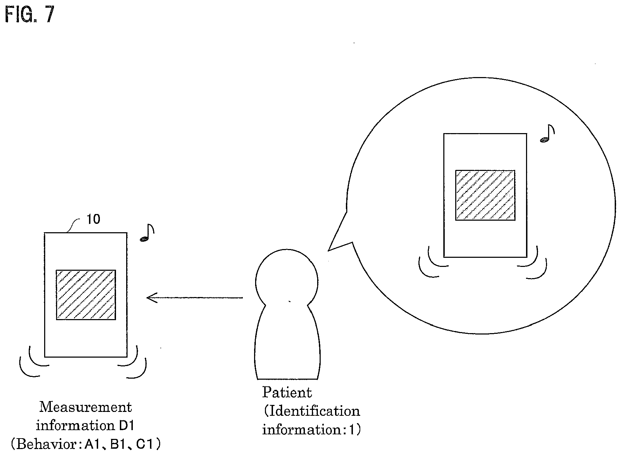

The following describes how the user, such as a healthcare worker or a measurement subject, identifies with behaviors a measuring apparatus to be used, with reference to FIGS. 6 and 7. FIG. 6 is a diagram illustrating a situation in which a patient as a measurement subject identifies the measuring apparatus 10 (measuring apparatus 10a) that is to be used for measurement, among a plurality of measuring apparatuses 10 (measuring apparatuses 10a, 10b, and 10c). FIG. 7 is a diagram illustrating a situation in which when a single measuring apparatus 10 is used for measurement for a plurality of measurement subjects, a patient as a measurement subject checks whether or not his/her own measurement information is set in the measuring apparatus 10.

The example of FIG. 6 shows a situation in which the plurality of measuring apparatuses 10a, 10b, and 10c are used in a hospital ward. Also in the example illustrated in FIG. 6, it is assumed that the recording unit 11 of each measuring apparatus has recorded therein only one behavior and one piece of identification information corresponding to the behavior. In such a case, if the healthcare worker or the measurement subject activates the measuring apparatuses, the above-described step S16 is then executed in each measuring apparatus, and therefore each measuring apparatus 10a, 10b, and 10c outputs the corresponding behavior. This allows the healthcare worker and the measurement subject to easily find the measuring apparatus 10a to be used.

Also, the example of FIG. 7 shows a situation in which a single measuring apparatus 10 is used for a plurality of patients. In the situation illustrated in FIG. 7, it is important that measurement information that is unique to each patient as a measurement subject is set in the measuring unit 15, and measurement results for each patient are accurately managed. The recording unit 11 of the measuring apparatus 10 thus has recorded therein a plurality of different behaviors and pieces of identification information corresponding to the respective behaviors.

As illustrated in FIG. 7, a patient who has input his/her own identification information into the input unit 16 can determine whether or not the behavior that the measuring apparatus 10 outputs matches the behavior that he/she has been input in advance. On the basis of this determination result, the patient can easily check whether his/her own measurement information is set in the measuring apparatus 10.

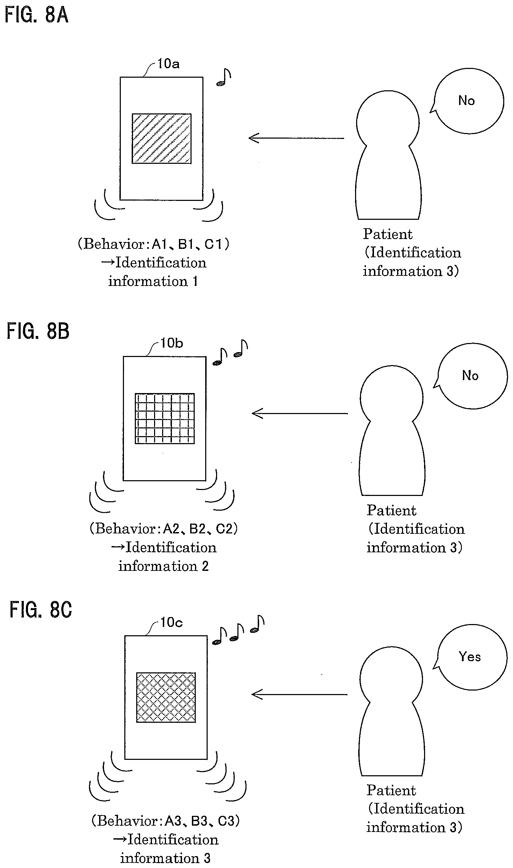

The following describes the case where the patient can select a behavior from the measuring apparatus 10 that corresponds to his/her own identification information based on the output behavior, with reference to FIGS. 8A to 8C. FIGS. 8A to 8C illustrate situations in which a patient as a measurement subject is performing selection operations based on behaviors from the measuring apparatus, and more specifically, FIGS. 8A to 8C illustrate states in which different behaviors are output.

In the examples of FIGS. 8A to 8C, the setting unit 12 of the measuring apparatus 10 causes the output unit 13 to output a plurality of different behaviors and sets, when any one of the behaviors is selected by the patient, the identification information associated with the selected behavior, as identification information of the patient. In this case, the setting unit 12 also sets the measurement information that is associated with the selected behavior as a measurement condition.

Specifically, as illustrated in FIGS. 8A to 8C, when the patient (who has identification information 3) gives a confirmation instruction for confirmation of the behavior via the button operation, the setting unit 12 causes the output unit 13 to sequentially output the behaviors stored in the table.

For example, as illustrated in FIG. 8A, the behavior (A1, B1, and C1) associated with the identification information 1 is first output (see FIG. 3). Next, the behavior (A2, B2, and C2) associated with identification information 2 is output, as illustrated in FIG. 8B. Next, the behavior (A3, B3, and C3) associated with identification information 3 is output, as illustrated in FIG. 8C.

In the example of FIG. 8, the identification information of the patient is the identification information 3, and therefore when the behavior (A3, B3, and C3) is output, the patient selects this behavior via a button operation. Next, the setting unit 12 sets the selected behavior as a behavior to be used, sets the identification information that is associated with the behavior, that is, the identification information 3 as identification information of the patient, and further sets the measurement information D1 that is associated with the selected behavior as a measurement condition (see FIG. 3). The aspect illustrated in FIGS. 8A to 8C is effective especially for the case where the patient has poor eyesight and cannot clearly view the identification information displayed on the display screen.

### Effects

As described above, the measuring apparatus 10 according to Embodiment 1 of the present invention can output behaviors that can be set, and enables the user such as a healthcare worker or a patient to select an identification number to be used on the basis of the output behaviors. According to the measuring apparatus 10, it is thus possible to easily and reliably correlate the identification information to be used and measured data with each other. This eliminates the need for the healthcare worker to perform a complicated operation as performed conventionally, thus suppressing a reduction in working efficiency.

In the measuring apparatus 10 of Embodiment 1 of the present invention, the behavior is defined by at least one of sound that is output by the measuring apparatus 10, screen display that is output by the measuring apparatus 10, light that is output by the measuring apparatus 10, and vibration that is output by the measuring apparatus 10.

Accordingly, setting, for example, different screen display depending on users of the measuring apparatus 10 thus enables a measurement subject, such as a patient or a healthcare worker, to easily check the behaviors by merely viewing the measuring apparatus 10. Also, setting different types of light, types of sound, and types of vibration that are to be output for each measurement subject by the measuring apparatus 10 enables even a measurement subject (patient) who has poor eyesight to easily check the behaviors. Further, combining two or more of the screen display, the type of light, the type of sound, and the type of vibration allows a measurement subject or a healthcare worker to check the behavior not only visually but also auditorily, making more accurate checking possible.

### Embodiment 2

Hereinafter, a measuring apparatus and a measuring system of Embodiment 2 of the present invention will be described with reference to the drawings. The measuring apparatus 10 of Embodiment 2 of the present invention is an apparatus configured to be able to intercommunicate with a communication terminal. Hereinafter, differences from Embodiment 1 will mainly be described.

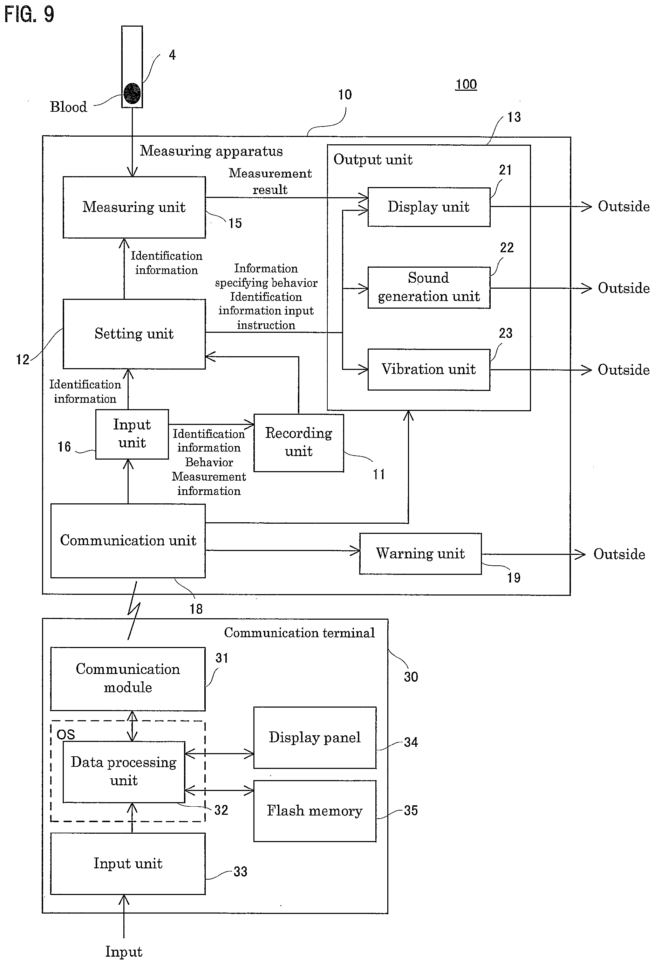

FIG. 9 is a block diagram illustrating a configuration of a measuring system 100 that includes the measuring apparatus 10 of Embodiment 2 of the present invention.

### System Configuration

As illustrated in FIG. 9, the measuring system 100 of Embodiment 2 of the present invention is provided with the measuring apparatus 10 and a communication terminal 30.

It is premised that the communication terminal 30 is carried by a patient as a measurement subject, a doctor or nurse as a healthcare worker, or the like. Also, the communication terminal 30 is a portable communication terminal that can communicate wirelessly with the measuring apparatus 10. Specific examples of the communication terminal 30 include a so-called "smartphone" on which a mobile device OS (Operating System) that can execute an application program is installed, and a tablet-type information terminal. The communication terminal 30 may also be a dedicated device for communicating with the measuring apparatus 10.

The communication terminal 30 includes a communication module 31, a data processing unit 32, an input unit 33, a display panel 34, and a flash memory 35.

The communication module 31 performs data transmission and reception with a communication unit 18 of the measuring apparatus 10. The data processing unit 32 is configured by executing an application program installed in the OS and outputs, via the communication module 31, a signal that instructs the communication unit 18 to operate, for example.

The input unit 33 is an input device such as a keyboard or a touch panel mounted on the communication terminal 30. The user of the communication terminal 30 can, for example, input data to and give an instruction to the measuring apparatus 10 via the input unit 33.

The display panel 34 is a display screen such as a liquid crystal display panel. The flash memory 35 has stored therein information for use in the communication terminal 30.

The main body 1 of the measuring apparatus 10 is provided with the communication unit 18, which can intercommunicate with the communication terminal 30, and a warning unit 19. The communication unit 18 is compatible with the communication system of the communication module 31 of the communication terminal 30. Examples of communication systems that are applicable in the present Embodiment 2 include a communication system utilizing a wireless LAN, and a communication system utilizing Bluetooth.

Particularly, when the communication system utilizing a wireless LAN is used, the measuring apparatus 10 and the communication terminal 30 that are associated in one-to-one correspondence in advance communicate with each other using an ad hoc mode, in which communication is performed directly without an intervening access point.

When a new behavior and identification information corresponding to the new behavior have been input from the communication terminal 30, the communication unit 18 can receive them, and output measurement information as well as the received behavior and identification information to the input unit 16. The input unit 16 outputs the new behavior, the identification information corresponding thereto, and the measurement information that were output by the communication unit 18 to the recording unit 11, and the recording unit 11 registers the new behavior, the identification information, and the measurement information.

By operating the communication terminal 30, therefore, the user can add the new behavior, the identification information corresponding thereto, and the measurement information to the recording unit 11, and can change already recorded behavior, identification information corresponding to the already recorded behavior, and measurement information.

Also, in the present Embodiment 2, the communication unit 18 can receive, from the communication terminal 30, a confirmation instruction for confirmation of the behavior, and notify the setting unit 12 of the confirmation instruction. In this case, similarly to the example in FIG. 8, the setting unit 12 causes the output unit 13 to sequentially output the behaviors stored in the table. In the present Embodiment 2, the communication unit 18 can also receive, from the communication terminal 30, the order of output of the behaviors, and the output unit 13, in this case, outputs the behaviors in the received order.

If any of the behaviors is selected, the setting unit 12 sets the identification information associated with the selected behavior as identification information to be used. In this case, the setting unit 12 also sets the measurement information associated with the selected behavior as a measurement condition.

The communication unit 18 can receive, from the communication terminal 30, a confirmation instruction for confirmation of the behavior corresponding to the set identification information. Upon receipt of the confirmation instruction from the communication terminal 30, the communication unit 18 causes the output unit 13 to output the currently set behaviors with the screen display, the sound, and the vibration, for example. The user thus can confirm the behavior that corresponds to his/her own identification information.

For the identification information in the setting unit 12, the communication unit 18 can also receive an identification number specification instruction for instructing the output unit 13 to output the behaviors in a predetermined order. When the communication unit 18 has received the identification number specification instruction, the setting unit 12 causes the output unit 13 to output the behaviors in a predetermined order. The user can thus easily and automatically check the behaviors in a desired order.

When the measuring apparatus 10 is in the operating state, and the communication unit 18 has received no signal from the communication terminal 30 for a threshold period of time or more for example, the communication unit 18 outputs a warning signal to the warning unit 19. Not only when having received no signal from the communication terminal 30 for a threshold period of time or more, but also when having detected change in the communication state with the communication terminal 30, the communication unit 18 may also output the warning signal to the warning unit 19.

For example, the communication unit 18 detects the radio field intensity of a signal transmitted from the communication terminal 30, and if this radio field intensity is less than a threshold, the communication unit 18 then determines that communication between the measuring apparatus and the communication terminal is difficult and outputs the warning signal to the warning unit 19. The communication unit 18 also detects the radio wave S/N ratio of the signal transmitted from the communication terminal 30, and if this radio wave S/N ratio is less than a threshold, the communication unit 18 similarly determines that communication between the measuring apparatus and the communication terminal is difficult and outputs the warning signal to the warning unit 19.

Furthermore, the communication unit 18 can output the warning signal to the warning unit 19 not only when having detected change in the communication state with the communication terminal 30, but also when having obtained distance information that indicates a distance between the measuring apparatus 10 and the communication terminal 30 using a global positioning system (GPS) or the like and detected, based on this distance, that the measuring apparatus 10 and the communication terminal 30 are separated from each other by a predetermined distance or more.

The warning unit 19 outputs a warning on the basis of the warning signal output from the communication unit 18. This warning can be made by, for example, outputting sound, or vibrating the measuring apparatus 10.

In addition, the present Embodiment 2 may have a configuration in which the warning is output from the communication terminal 30. For example, a configuration is also possible in which, when having detected that the measuring apparatus 10 and the communication terminal 30 are separated from each other by a predetermined distance or more, the communication unit 18 of the measuring apparatus 10 transmits the warning signal to the communication terminal 30 so that the communication terminal 30 outputs a warning sound or the like.

Note that configurations of the measuring apparatus 10 in Embodiment 2 of the present invention that have not been described above are similar to the corresponding configurations of the measuring apparatus 10 of Embodiment 1 illustrated in FIG. 2, and thus descriptions thereof will be omitted.

### Apparatus Operation

Now, operation of the measuring system 100 of Embodiment 2 of the present invention will be described. First, the measuring apparatus 10 according to the present Embodiment 2 performs preliminary preparation processing similar to the preliminary preparation processing of Embodiment 1 illustrated in FIG. 4. The measuring apparatus 10 according to the present Embodiment 2 also performs measurement processing similar to the measurement processing of Embodiment 1 illustrated in FIG. 5. Accordingly, in the following description, FIGS. 4 and 5 may suitably be referenced.

However, in the present Embodiment 2, the input in step S1 of the preliminary preparation processing illustrated in FIG. 4 is executed by outputting information from the communication terminal 30 to the communication unit 18. The setting of the operating state in step S11 and the input of identification information in step S14 of the measurement processing illustrated in FIG. 5 may also be executed by outputting information from the communication terminal 30 to the communication unit 18.

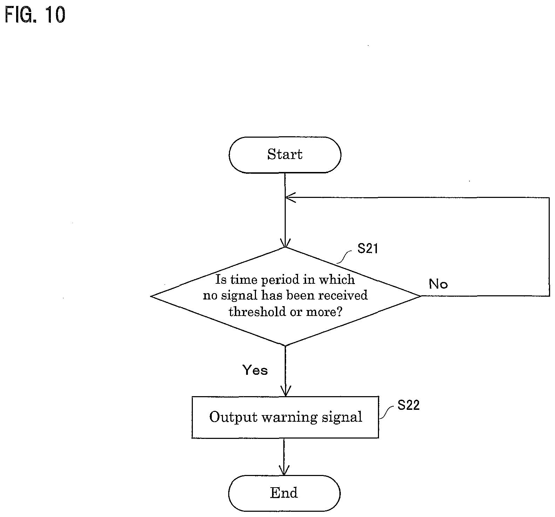

Further in the present Embodiment 2, the measuring apparatus 10 can execute warning processing for preventing the measuring apparatus 10 from being misplaced, in parallel to the measurement processing illustrated in FIG. 5. FIG. 10 is a flowchart illustrating a flow of the warning processing performed in the measuring apparatus of Embodiment 2 of the present invention.

As illustrated in FIG. 10, in the case where the measuring apparatus 10 is in the operating state, the communication unit 18 first determines whether or not a period of time in which it has received no signal from the communication terminal 30 is a threshold or greater (step S21).

If the result of the determination in step S21 is that the period of time in which the communication unit 18 has received no signal from the communication terminal 30 is less than the threshold, the communication unit 18 executes step S21 again after a set period of time has elapsed. On the other hand, if the result of the determination in step S21 is that the period of time in which the communication unit 18 has received no signal from the communication terminal 30 is the threshold or greater, the communication unit 18 outputs the warning signal in order to instruct the warning unit 19 to give a warning (step S22).

When step S22 has been executed, the warning unit 19 outputs a warning in accordance with the warning signal output from the communication unit 18. This enables the user such as a healthcare worker or a measurement subject to find out that communication between the measuring apparatus 10 and the communication terminal 30 is interrupted.

### Effects

As described above, the measuring apparatus 10 of Embodiment 2 of the present invention is provided with the communication unit 18 that can intercommunicate with the communication terminal 30, enabling the measuring apparatus 10 to be made easy to use such that the measuring apparatus 10 is activated, for example, by operation of the communication terminal 30.

Also, in the measuring apparatus 10 of Embodiment 2 of the present invention, upon input of a new behavior and identification information corresponding thereto from the communication terminal 30, the communication unit 18 receives the new behavior and the identification information. When the communication unit 18 has received the input of the new behavior and the identification information corresponding thereto, the recording unit 11 records the received new behavior and identification information corresponding thereto.

Accordingly, the user of the measuring apparatus 10 can add a new behavior output from the measuring apparatus 10 or change existing behavior by operating the communication terminal 30.

In the measuring apparatus 10 of Embodiment 2 of the present invention, the communication unit 18 also receives, from the communication terminal 30, a confirmation instruction for confirmation of the behavior that corresponds to the identification information of the user of the measuring apparatus 10. When the communication unit 18 has received the confirmation instruction, the output unit 13 outputs the behavior corresponding to the identification information of the user of the measuring apparatus 10.

This makes it possible for the user to easily confirm the behavior that corresponds to his/her own identification information.

In the measuring apparatus 10 of Embodiment 2 of the present invention, the communication unit 18 can also output the warning signal to the warning unit 19 when having detected change in the communication state, such as when having received no signal from the communication terminal 30 for a threshold period of time or more, when the signal transmitted from the communication terminal 30 has a radio field intensity of not more than a threshold or when the signal transmitted from the communication terminal 30 has a radio wave S/N ratio of not more than a threshold. Also, the warning unit 19 outputs a warning in accordance with the warning signal output from the communication unit 18.

Since a warning will be given if the communication terminal 30 and the measuring apparatus 10 are separated from each other and no signal is thus transmitted and received therebetween, it is possible to prevent the measuring apparatus 10 from being misplaced.

As described above, the present invention is useful in the field of medical devices, in particular, small-sized portable medical devices.

### Descriptions of Reference Numerals

- 1: Main body
- 2: Display screen
- 3: Sensor insertion opening
- 4: Sensor
- 5: Button
- 10: Measuring apparatus
- 11: Recording unit
- 12: Setting unit
- 13: Output unit
- 15: Measuring unit
- 16: Input unit
- 18: Communication unit
- 19: Warning unit
- 21: Display unit
- 22: Sound generation unit
- 23: Vibration unit
- 30: Communication terminal
- 31: Communication module
- 32: Data processing unit
- 33: Input unit
- 34: Display panel
- 35: Flash memory
- 100: Measuring system

## Claims

1. A measuring apparatus (10) for measuring the condition of a measurement subject, the measuring apparatus (10) comprising:
a measuring unit (15) for obtaining a measurement result;
an input unit (16) for receiving input of information and for outputting the received information to a setting unit (12);
a recording unit (11) configured to record a plurality of behaviors that can be set in the measuring apparatus, and pieces of user-specific or medical-facility-specific identification information that are associated with the plurality of behaviors;
the setting unit (12) configured to specify a piece of measurement subject-specific identification information that is correlated with a measurement result in accordance with external input, and to set the specified piece of identification information and the behavior recorded by the recording unit associated with the specified piece of identification information, as identification information and a behavior that are to be used; and
an output unit (13) configured to output the behavior set by the setting unit (12);
wherein the behaviors are (a) signals to allow a user to identify the measuring apparatus (10) from among a plurality of such measuring apparatuses, or (b) signals to allow a user to determine whether or not the measuring apparatus (10) has been set with measurement information of the user.

2. The measuring apparatus (10) according to claim 1,
wherein the behaviors are each defined by at least one of sound that is output by the measuring apparatus (10), screen display that is output by the measuring apparatus (10), light that is output by the measuring apparatus (10), and vibration that is output by the measuring apparatus (10).

3. The measuring apparatus (10) according to claim 1,
wherein the setting unit (12) is configured to cause the output unit (13) to output the plurality of different behaviors and to specify, when any of the behaviors is selected through external input, a piece of identification information that is associated with the selected behavior as the identification information correlated with the measurement result.

4. The measuring apparatus (10) according to any one of claims 1 to 3,
further comprising a communication unit (18) configured to be able to intercommunicate with a communication terminal.

5. The measuring apparatus (10) according to claim 4,
wherein the communication unit (18) is configured to receive a new behavior and identification information that is associated with the new behavior, upon input of said new behavior and said identification information from the communication terminal (30), and
the recording unit (11) is configured to record said new behavior and said identification information associated with the new behavior when received by the communication unit (18).

6. The measuring apparatus (10) according to claim 4 or 5,
wherein the communication unit (18) is configured to receive from the communication terminal (30) a confirmation instruction for confirmation of the behavior associated with the identification information, and
the output unit (13) is configured to output the behavior associated with the identification information when the communication unit (18) has received the confirmation instruction.

7. The measuring apparatus (10) according to any one of claims 4 to 6,
further comprising a warning unit (19) configured to output a warning,
wherein the communication unit (18) is configured to cause the warning unit (19) to output a warning, when having detected a change in a communication state with the communication terminal (30).

8. A measuring system (100) comprising:
a measuring apparatus (10) according to any of claims 4 to 7, and a communication terminal (30).

## Patentansprüche

1. Messvorrichtung (10) zum Messen des Zustands eines Messgegenstands, wobei die Messvorrichtung (10) Folgendes aufweist:
eine Messeinheit (15) zum Erhalten eines Messergebnisses;
eine Eingabeeinheit (16) zum Empfangen einer Informationseingabe und zum Ausgeben der empfangenen Informationen an eine Einstelleinheit (12);
eine Aufzeichnungseinheit (11), die zum Aufzeichnen mehrerer Verhalten, die in der Messvorrichtung eingestellt werden können, und von benutzerspezifischen oder für eine medizinische Einrichtung spezifischen Identifikationsinformationen, die mit den mehreren Verhalten assoziiert sind, konfiguriert ist;
die Einstelleinheit (12), die zum Vorgeben einer messgegenstandsspezifischen Identifikationsinformation, die gemäß externer Eingabe mit einem Messergebnis korreliert ist, und zum Einstellen der vorgegebenen Identifikationsinformation und des von der Aufzeichnungseinheit aufgezeichneten Verhaltens, das mit der vorgegebenen Identifikationsinformation assoziiert ist, als Identifikationsinformation und ein Verhalten, die zu verwenden sind, konfiguriert ist; und
eine Ausgabeeinheit (13), die zum Ausgeben des von der Einstelleinheit (12) eingestellten Verhaltens konfiguriert ist;
wobei die Verhalten (a) Signale sind, um einem Benutzer die Identifizierung der Messvorrichtung (10) unter mehreren derartigen Messvorrichtungen zu ermöglichen, oder (b) Signale, um einem Benutzer das Bestimmen zu ermöglichen, ob die Messvorrichtung (10) mit Messinformationen des Benutzers eingestellt wurde.

2. Messvorrichtung (10) nach Anspruch 1,
wobei die Verhalten jeweils von wenigstens einem der Folgenden definiert werden: Ton, der von der Messvorrichtung (10) ausgegeben wird, Bildschirmanzeige, die von der Messvorrichtung (10) ausgegeben wird, Licht, das von der Messvorrichtung (10) ausgegeben wird, und Vibration, die von der Messvorrichtung (10) ausgegeben wird.

3. Messvorrichtung (10) nach Anspruch 1,
wobei die Einstelleinheit (12) zum Veranlassen der Ausgabeeinheit (13) zum Ausgeben der mehreren verschiedenen Verhalten und, wenn eines der Verhalten durch externe Eingabe ausgewählt wird, zum Vorgeben einer Identifikationsinformation, die mit dem ausgewählten Verhalten assoziiert ist, als die mit dem Messergebnis korrelierten Identifikationsinformationen konfiguriert ist.

4. Messvorrichtung (10) nach einem der Ansprüche 1 bis 3,
die ferner eine Kommunikationseinheit (18) aufweist, die konfiguriert ist, um mit einem Kommunikationsendgerät vernetzt sein zu können.

5. Messvorrichtung (10) nach Anspruch 4,
wobei die Kommunikationseinheit (18) bei Eingabe des genannten neuen Verhaltens und der genannten Identifikationsinformationen von dem Kommunikationsendgerät (30) zum Empfangen eines neuen Verhaltens und von Identifikationsinformationen, die mit dem neuen Verhalten assoziiert sind, konfiguriert ist, und
die Aufzeichnungseinheit (11) zum Aufzeichnen des genannten neuen Verhaltens und der genannten Identifikationsinformationen, die mit dem neuen Verhalten assoziiert sind, wenn sie von der Kommunikationseinheit (18) empfangen werden, konfiguriert ist.

6. Messvorrichtung (10) nach Anspruch 4 or 5,
wobei die Kommunikationseinheit (18) zum Empfangen einer Bestätigungsanweisung zur Bestätigung des Verhaltens, das mit den Identifikationsinformationen assoziiert ist, von dem Kommunikationsendgerät (30) konfiguriert ist, und
die Ausgabeeinheit (13) zur Ausgabe des Verhaltens, das mit den Identifikationsinformationen assoziiert ist, wenn die Kommunikationseinheit (18) die Bestätigungsanweisung empfangen hat, konfiguriert ist.

7. Messvorrichtung (10) nach einem der Ansprüche 4 bis 6,
die ferner eine Warneinheit (19) aufweist, die zur Ausgabe einer Warnung konfiguriert ist,
wobei die Kommunikationseinheit (18) konfiguriert ist, um die Warneinheit (19) zur Ausgabe einer Warnung zu veranlassen, wenn sie eine Änderung des Kommunikationszustands mit dem Kommunikationsendgerät (30) erkannt hat.

8. Messsystem (100), das Folgendes aufweist:
eine Messvorrichtung (10) nach einem der Ansprüche 4 bis 7 und ein Kommunikationsendgerät (30).

## Revendications

1. Appareil de mesure (10) servant à mesurer l'état d'un sujet soumis à la mesure, l'appareil de mesure (10) comportant :
une unité de mesure (15) servant à obtenir un résultat de la mesure ;
une unité d'entrée (16) servant à recevoir une entrée sous forme d'informations et à émettre en sortie l'information reçue à destination d'une unité de réglage (12) ;
une unité d'enregistrement (11) configurée pour enregistrer une pluralité de comportements qui peuvent être réglés dans l'appareil de mesure, et des informations d'identification spécifiques à l'utilisateur ou spécifiques à l'installation médicale qui sont associées à la pluralité de comportements ;
l'unité de réglage (12) étant configurée pour spécifier une information d'identification spécifique au sujet soumis à la mesure qui est corrélée à un résultat de la mesure en fonction de l'entrée externe, et pour régler l'information d'identification spécifiée et le comportement enregistré par l'unité d'enregistrement associé à l'information d'identification spécifiée, comme information d'identification et comportement qui doivent être utilisés ; et
une unité de sortie (13) configurée pour émettre en sortie le comportement réglé par l'unité de réglage (12) ;
dans lequel les comportements sont (a) des signaux qui permettent à un utilisateur d'identifier l'appareil de mesure (10) parmi une pluralité de tels appareils de mesure, ou (b) des signaux qui permettent à un utilisateur de déterminer si oui ou non l'appareil de mesure (10) a été réglé avec des informations de mesure de l'utilisateur.

2. Appareil de mesure (10) selon la revendication 1,
dans lequel les comportements sont chacun définis par au moins l'un parmi un son qui est émis en sortie par l'appareil de mesure (10), un affichage sur écran qui est émis en sortie par l'appareil de mesure (10), une lumière qui est émise en sortie par l'appareil de mesure (10), et des vibrations qui sont émises en sortie par l'appareil de mesure (10).

3. Appareil de mesure (10) selon la revendication 1,
dans lequel l'unité de réglage (12) est configurée pour amener l'unité de sortie (13) à émettre en sortie la pluralité de différents comportements et à spécifier, quand l'un quelconque des comportements est sélectionné par une entrée externe, une information d'identification qui est associée au comportement sélectionné comme information d'identification corrélée au résultat de la mesure.

4. Appareil de mesure (10) selon l'une quelconque des revendications 1 à 3,
comportant par ailleurs une unité de communication (18) configurée à des fins d'intercommunication avec un terminal de communication.

5. Appareil de mesure (10) selon la revendication 4,
dans lequel l'unité de communication (18) est configurée pour recevoir un nouveau comportement et une information d'identification qui est associée au nouveau comportement, lors de l'entrée dudit nouveau comportement et de ladite information d'identification en provenance du terminal de communication (30), et
l'unité d'enregistrement (11) est configurée pour enregistrer le nouveau comportement et ladite information d'identification associée au nouveau comportement dès réception par l'unité de communication (18).

6. Appareil de mesure (10) selon la revendication 4 ou la revendication 5,
dans lequel l'unité de communication (18) est configurée pour recevoir, en provenance du terminal de communication (30), une instruction de confirmation servant à confirmer le comportement associé à l'information d'identification, et
l'unité de sortie (13) est configurée pour émettre en sortie le comportement associé à l'information d'identification quand l'unité de communication (18) a reçu l'instruction de confirmation.

7. Appareil de mesure (10) selon l'une quelconque des revendications 4 à 6,
comportant par ailleurs une unité d'avertissement (19) configurée pour émettre en sortie un avertissement,
dans lequel l'unité de communication (18) est configurée pour amener l'unité d'avertissement (19) à émettre en sortie un avertissement, lors de la détection d'un changement d'un état de communication avec le terminal de communication (30).

8. Système de mesure (100) comportant :
un appareil de mesure (10) selon l'une quelconque des revendications précédentes 4 à 7, et un terminal de communication (30).
